# EUROPÄISCHE PATENTANMELDUNG

(11) **EP 3 796 331 A1**
(43) Veröffentlichungstag der Anmeldung: **24.03.2021**
(21) Anmeldenummer: 19198939.1
(22) Anmeldetag: 23.09.2019
(51) Int. Cl.: G16H 40/20, G06Q 50/00, G16H 40/67, B25J 9/16, G05D 1/00, G05D 1/02

(54) **SYSTEM ZUR KOOPERATIVEN BEWEGUNGSSTEUERUNG UND/ODER BEWEGUNGSÜBERWACHUNG VON MOBILEN MEDIZINKOMPONENTEN**

(71) Anmelder: Siemens Healthcare GmbH, 91052 Erlangen (DE)
(72) Erfinder: Deinlein, Andreas, 95447 Bayreuth (DE)

(57) **Zusammenfassung**

In einem Aspekt betrifft die Erfindung ein System zur kooperativen Bewegungssteuerung und/oder Bewegungsüberwachung von mobilen Medizinkomponenten in einer medizinischen Einrichtung. Die medizinische Einrichtung, wie z.B. ein Krankenhaus weist eine Vielzahl an Einrichtungseinheiten auf, umfassend eine Vielzahl an mobilen Medizinkomponenten, die über eine Kommunikationsverbindung zueinander in Verbindung stehen und kontinuierlich kommunizieren, um über ein Kommunikationsprotokoll kooperativ die Bewegung jeder mobilen Medizinkomponente der Vielzahl an mobilen Medizinkomponenten in der medizinischen Einrichtung und/oder in den Einrichtungseinheiten zu überwachen und/oder zu steuern.

## Beschreibung

Die Erfindung betrifft ein System zur kooperativen Bewegungsüberwachung und/oder Bewegungssteuerung von mobilen Medizinkomponenten in einer medizinischen Einrichtung mit einer Vielzahl an Einrichtungseinheiten. Ferner betrifft die Erfindung eine entsprechende mobile Medizinkomponente, ein Verfahren zum Betreiben des Systems und ein Verfahren zum Betreiben der Medizinkomponente, sowie ein Computerprogramm.

In einer medizinischen Einrichtung, beispielsweise in einem Krankenhaus, werden Medizinkomponenten (z.B. bildgebende Geräte) von unterschiedlichen Herstellern für unterschiedliche Einsatzzwecke, wie z.B. für eine medizinische Diagnose, bereitgestellt. Die Medizinkomponenten werden unabhängig voneinander spezifiziert, entwickelt und konstruiert. Dabei handelt es sich in den meisten Fällen um proprietäre Speziallösungen. Diese proprietären Speziallösungen können Kommunikationsverfahren, Kommunikationsschnittstellen und/oder eine Datenverarbeitung aufweisen, die untereinander nicht kompatibel sind und somit keine Kommunikation oder Datenaustausch zwischen den verschiedenen Medizinkomponenten ermöglichen. Hierbei ist von Nachteil, dass diese Medizinkomponenten nicht miteinander kooperieren können, um einen gemeinsamen Ablauf in einem Krankenhaus zu ermöglichen. Dies schränkt die Möglichkeit einer Automatisierung in einer medizinischen Einrichtung ein und verhindert dadurch einen effizienten Workflow in der medizinischen Einrichtung.

Lösungen sehen die Verwendung von speziell ausgestalteten Schnittstellen vor, in denen beispielsweise ein AngiographieGerät mit einem Computertomografiegerät Daten austauschen kann. Dies setzt voraus, dass eine medizinische Komponente, beispielsweise das Angiographiegerät der Master ist und die notwendige Schnittstelle zur Kommunikation implementiert. Diese stellt aber eine spezielle Schnittstelle dar, über welche nur diese beiden zuvor genannten medizinischen Komponenten kommunizieren und Daten austauschen können und somit zusammen einen speziell abgestimmten klinischen Workflow realisieren. Diese im speziellen von einem Gerät zu einem weiteren Gerät bereitgestellte Kommunikation, ermöglicht aber nicht die Aufnahme weiterer teilnehmender Geräte, um somit einen ganzheitlichen klinischen Workflow aufzubauen bzw. bereitzustellen.

Die fehlende bzw. eingeschränkte Kommunikation und der fehlende Datenaustausch werden noch problematischer, wenn in einer medizinischen Einrichtung mobile motorisch fahrbare Medizinkomponenten (medizinische fahrerloses Transportfahrzeug - medical AGVs - Automated Guided Vehicles) eingesetzt werden. Die medizinischen fahrerlosen Transportfahrzeuge sind flurgebundene medizinische Komponenten mit einem eigenen Fahrantrieb. Diese können nach Anforderung automatisch gesteuert und berührungslos geführt werden. Um einen effizienten Einsatz und Ablauf und ohne Kollisionen und/oder Störungen der medizinisch fahrerlosen Transportfahrzeuge zu ermöglichen ist eine Verständigung und insbesondere eine Kooperation zwischen den einzelnen medizinischen fahrerlosen Transportfahrzeugen innerhalb der medizinischen Einrichtung notwendig.

Es besteht daher ein Bedarf an einem Mechanismus zur kooperativen Bewegungsüberwachung und/oder Bewegungssteuerung von mobilen Medizinkomponenten in einer medizinischen Einrichtung mit einer Vielzahl an Einrichtungseinheiten. Ausgehend vom aufgezeigten Stand der Technik und dem sich daraus ergebenden Bedarf, hat sich die vorliegende Erfindung zur Aufgabe gestellt, eine Lösung zu schaffen, die die im Stand der Technik bekannten Nachteile zu mindestens teilweise überwindet.

Diese Aufgabe wird durch die nebengeordneten beiliegenden Patentansprüche gelöst, insbesondere durch ein System, eine mobile Medizinkomponente, ein Verfahren und ein Computerprogramm.

Gemäß einem ersten Aspekt betrifft die Erfindung ein System zur kooperativen Bewegungsüberwachung und/oder Bewegungssteuerung von mobilen Medizinkomponenten (z.B. fahrbare medizinische, z.B. bildgebende, Geräte) in einer medizinischen Einrichtung. Die medizinische Einrichtung, wie z.B. eine Klinik, ist für das System in mehrere Einrichtungseinheiten, wie z.B. Räume, Bereiche (Operationsraum, Sterilbereich), gegliedert oder strukturiert. Das System umfasst und steuert eine Vielzahl an mobilen Medizinkomponenten, die über eine Kommunikationsverbindung miteinander in Datenaustausch stehen und kontinuierlich kommunizieren, um über ein Kommunikationsprotokoll kooperativ die Bewegung jeder mobilen Medizinkomponente der Vielzahl an mobilen Medizinkomponenten in der medizinischen Einrichtung und/oder in den Einrichtungseinheiten zu überwachen und/oder zu steuern.

In vorteilhafter Weise wird durch die vorliegende Erfindung die Bewegung jeder mobilen Medizinkomponente kooperativ überwacht und/oder gesteuert. Im Sinne der vorliegenden Erfindung ist unter kooperativer Überwachung und/oder Steuerung eine zusammenwirkende bzw. zusammenarbeitende Überwachung und/oder Steuerung zu verstehen, so dass jede der Vielzahl an mobilen Medizinkomponenten zum Überwachen und Steuern ausgebildet ist. Jede mobile Medizinkomponente der Vielzahl an mobiler Medizinkomponenten verfügt über eine eigene Prozessoreinheit und somit über eine eigene dezentrale Intelligenz, über die eine Überwachung und/oder Steuerung der mobilen Medizinkomponenten erfolgen kann. Jede mobile Medizinkomponente kann Navigationssignale der weiteren mobilen Medizinkomponenten empfangen und über die eigene Prozessoreinheit (dezentrale Intelligenz) Navigationssignale erzeugen und bereitstellen. Die erzeugten Navigationssignale können zur Steuerung der navigationssignalerzeugenden mobilen Medizinkomponente und/oder für die weiteren mobilen Medizinkomponenten bereitgestellt werden. Somit wird eine autonome Bewegung mehrerer mobiler Medizinkomponenten in einer medizinischen Einrichtung, insbesondere in einer automatisierten medizinischen Einrichtung ohne eine zentrale Intelligenz ermöglicht. Insbesondere kann eine gemeinschaftliche Bewegungsüberwachung und/oder Bewegungssteuerung erfolgen. Die Bewegungsüberwachung und/oder Bewegungssteuerung wird einrichtungsspezifisch und vorzugsweise einrichtungseinheitenspezifisch ausgeführt. Dies bedeutet, dass die Bewegungssteuerung und/oder Überwachung der jeweiligen mobilen Medizinkomponente in Abhängigkeit davon erfolgt, in welcher Einrichtungseinheit sich die Komponente befindet (IST-Position) oder bewegen soll (angeforderte SOLLPosition). Umfasst z.B. eine erste Einrichtungseinheit eine Sperrzone und eine zweite Einrichtungseinheit keine Sperrzone, so erfolgt die Bewegungssteuerung für die jeweilige Komponente in der ersten Einrichtungseinheit anders und abweichend von der zweiten Einrichtungseinheit, nämlich unter Berücksichtigung der Sperrzone.

Weiterhin vorteilhaft ist, dass mobile Medizinkomponenten, die nicht die notwendige Sensoreinheit zur Detektion der Route oder einer Karte umfassen, über die kontinuierliche Kommunikationsverbindung ein Update über Änderungen in den Einrichtungseinheiten oder der Karte empfangen können. Diese Informationen können durch andere mobile Medizinkomponenten mit der entsprechenden Sensoreinheit erzeugt und bereitgestellt werden.

Im Sinne der vorliegenden Erfindung umfasst eine medizinische Einrichtung eine Einrichtung, in welcher medizinische Untersuchungen und/oder Behandlungen unter Verwendung von Medizinkomponenten durchgeführt werden. Die medizinische Einrichtung kann beispielsweise ein Krankenhaus, eine Ambulanz, ein medizinisches Forschungslabor, eine Krankenstation und/oder eine Poliklinik umfassen oder als solche ausgebildet sein. Die bespielhafte Auflistung stellt dabei keine abschließende Auflistung bzw. Einschränkung dar. Vielmehr sind weitere Einrichtungen denkbar, in denen medizinische Untersuchungen und/oder Behandlungen stattfinden können. Die medizinische Einrichtung kann eine Vielzahl ein Einrichtungseinheiten umfassen. Eine Einrichtungseinheit kann beispielsweise einen Raum als abgegrenzten Bereich, einen Korridor, eine Treppe, einen Aufzug als Bewegungsbereich und/oder einen nicht abgegrenzten Bereich (freier Bereich) umfassen.

Weiterhin ist im Sinne der vorliegenden Erfindung unter einer mobilen Medizinkomponente eine Komponente zu verstehen, welche über einen eigenen Antrieb und eine eigene Energieversorgung verfügt. Die mobile Medizinkomponente kann z.B. eine mobile Liege zum Ablegen von Patienten für die Untersuchung umfassen. Zudem kann eine mobile Medizinkomponente ein Untersuchungsgerät, ein Gerät zur Ausführung eines bildgebenden Verfahrens, beispielsweise CT, MRT, Röntgen oder Ultraschall umfassen. Weiterhin umfassen medizinische Komponenten auch die medizinischen oder klinischen Geräte, welche zur Diagnose, Heilung und/oder Lebenserhaltung eingesetzt werden.

Gemäß der Erfindung stehen die mobilen Medizinkomponenten über zumindest eine Kommunikationsverbindung zueinander in Verbindung. Gemäß einer bevorzugten Ausführungsform der vorliegenden Erfindung wird die Kommunikationsverbindung über eine Wi-Fi-Verbindung (WLAN) hergestellt. Wi-Fi ist eine Technologie für die drahtlose lokale Vernetzung von Geräten, beispielsweise von mobilen Medizinkomponenten, die auf den Standards IEEE 802.11 basieren. Eine Wi-Fi-Verbindung ermöglicht eine drahtlose Verbindung eines Gerätes und/oder einer mobilen Medizinkomponente mit einem Kommunikationsgateway mit einer hohen Bandbreite von 72 bis 9608 Mbit/s, je nach Version (Wi-Fi4, Wi-Fi5, Wi-Fi5) des verwendeten Standards. Daher können Daten von der mobilen Medizinkomponente zum Kommunikationsgateway und zu den weiteren mobilen Medizinkomponenten übertragen werden.

Gemäß einer weiteren bevorzugten Ausführungsform der vorliegenden Erfindung wird die Kommunikationsverbindung durch eine Bluetooth-Verbindung hergestellt. Die Bluetooth-Verbindung ist ein drahtloser Technologiestandard für den Datenaustausch zwischen dem Kommunikationsgateway und der mobilen Medizinkomponente über eine kurze Distanz unter Verwendung kurzwelliger UHF-Funkwellen von 2.400 bis 2.485 GHz. Somit können Daten von der mobilen Medizinkomponente zum Kommunikationsgateway und zu den weiteren mobilen Medizinkomponenten übertragen werden.

Gemäß einer weiteren Ausführungsform der vorliegenden Erfindung wird die Kommunikationsverbindung durch eine Bluetooth-Niederenergieverbindung (BLE) hergestellt. Bluetooth Low Energy, ist eine drahtlose Personal Area Network Technologie, die verwendet werden kann, um mobile Geräte in einer Umgebung von etwa 10 Metern zu verbinden. Bluetooth Low Energy hat einen deutlich niedrigeren Stromverbrauch und niedrigere Kosten bei gleicher Reichweite. Somit können Daten von der mobilen Medizinkomponente zum Kommunikationsgateway und zu den weiteren mobilen Medizinkomponenten übertragen werden.

Gemäß einer weiteren Ausführungsform der vorliegenden Erfindung kann die Kommunikationsverbindung über eine Infrarotverbindung hergestellt werden. Eine Infrarotverbindung ermöglicht eine drahtlose Datenübertragung für eine maximale Reichweite über den letzten Meter nach dem Point-and-Shoot-Prinzip. Die Infrarot-Kommunikation bietet eine physikalisch sichere Datenübertragung innerhalb einer Sichtlinie und weist eine sehr niedrige Bitfehlerrate auf, wodurch diese sehr effizient ist. Über eine Infrarotverbindung können Daten von der einer mobilen Medizinkomponente direkt zu den weiteren mobilen Medizinkomponenten in der medizinischen Einrichtung übertragen werden, wenn diese sich in einer Sichtlinie aufhalten und/oder einander passieren.

Über die Kommunikationsverbindungen erfolgt eine kontinuierliche Kommunikation zwischen den mobilen Medizinkomponenten in der medizinischen Einrichtung. Hierbei sind die mobilen Medizinkomponenten in Abhängigkeit der Konfiguration und Verwendung (Master oder Slave) derart ausgebildet, dass über die Kommunikationsverbindung ein stetes Abhören zum Empfang (Slave) von neuen Navigationssignalen und/oder Daten erfolgt. Eine als Master ausgebildete mobile Medizinkomponente kann stetig Navigationssignale und/oder Daten senden.

In einer Ausführungsform kann die kontinuierliche Kommunikation eine periodisch kontinuierliche Kommunikation umfassen. Somit können die verwendete Bandbreite und Datenlast reduziert werden.

Gemäß einer Ausführungsform der Erfindung ist das Kommunikationsprotokoll der mobilen Medizinkomponenten als Master-Slave-Protokoll ausgebildet. Hierbei ist eine mobile Medizinkomponente als Master angewählt und die weiteren mobilen Medizinkomponenten sind als Slave angewählt. In vorteilhafter Weise erfolgt somit ein Handshake zwischen den beteiligten Medizinkomponenten, in welcher der Status des Masters an eine andere Medizinkomponente übergeben werden kann. Nur die Medizinkomponente, welche den Status des Masters hat, darf andere an dem aktuellen Workflow beteiligte Medizinkomponenten fernsteuern. Somit können die Zusammenarbeit und Steuerung, sowie der Workflow der Medizinkomponenten auf Verfügbarkeit optimiert werden.

In einer Ausführungsform kann die Kommunikation zwischen den Medizinkomponenten über ein Publish-Subscribe-Protokoll (DDS) gemäß dem Robot Operating System (ROS2) erfolgen. Hier kann jede Komponente mit jeder anderen kommunizieren ohne dass die Notwendigkeit eines "Masters" für die Steuerung der Kommunikation besteht. In vorteilhafter Weise ist das Kommunikationsprotokoll auch bei einem Ausfall einer oder mehrerer Medizinkomponenten funktionstüchtig. Ein Ausfall kann hierbei auch den Master umfassen. Diese würde sogar weiterhin das Hinzufügen von weiteren Medizinkomponenten über, beispielsweise "Plug&Play" erlauben.

Die für eine Untersuchung notwendige mobile Medizinkomponente kann über ein Anforderungssignal an einer Auswahl-Benutzerschnittstelle von einem Nutzer, beispielsweise einem behandelnden Arzt ausgewählt werden und als sogenannte Masterkomponente festgelegt werden. Der Status als Master kann für die Zeit der Untersuchung festgelegt werden und die weiteren für die Untersuchung notwendigen mobilen Medizinkomponenten bekommen den Status des Slave zugeordnet. Der Status kann in einer Statusnachricht gespeichert und/oder übertragen werden. Die Statusnachricht kann weitere Informationen über die mobile Medizinkomponente umfassen, wie z.B. deren aktuelle Position, die Information, ob sie angefordert wurde, ob für sie ein Freigabesignal erlassen wurde, wie häufig sie angefordert wird etc. Jede mobile Medizinkomponente kann den jeweils ihr zugeordneten Status in der Statusnachricht über eine Benutzerschnittstelle, beispielsweise über ein Display, anzeigen. Die Masterkomponente kann Navigationssignale bereitstellen, um die Slave Komponenten fernzusteuern. In vorteilhafter Weise können somit eine effiziente und optimierte Bewegung und Bereitstellung der mobilen Medizinkomponenten erfolgen. Zudem erfolgt die Überwachung und Steuerung derart, dass es zu keiner Kollision zwischen den mobilen Medizinkomponenten kommt.

In einer weiteren Ausführungsform kann die Rolle des Masters von einer mobilen Medizinkomponente auf eine andere mobile Medizinkomponente abgegeben werden. Dies ist dann von Vorteil, wenn während einer Untersuchung und/oder Behandlung ein Wechsel der Medizinkomponente erfolgt. Hierdurch wird die Sicherheit gegenüber der Bewegung der mobilen Medizinkomponenten erhöht.

Gemäß einer weiteren Ausführungsform der Erfindung umfasst eine Einrichtungseinheit der Vielzahl an Einrichtungseinheiten der medizinischen Einrichtung wenigstens zwei getrennte aufgabenspezifische Zonen. Eine Einrichtungseinheit, beispielsweise ein Behandlungsraum umfasst wenigstens zwei getrennte aufgabenspezifische Zonen. Die aufgabenspezifischen Zonen stellen festgelegte Bereiche dar. In diesen Bereichen dürfen nur die der Zone zugewiesene Aufgaben ausgeführt werden. Die aufgabenspezifischen Zonen können über Koordinaten, Marker und/oder Begrenzungsmarkierungen definiert werden. Die Koordinaten, Marker und/oder Begrenzungsmarkierungen können über eine Sensoreinheit erfasst werden. In einer Ausführungsform können die Koordinaten, Marker und/oder Begrenzungsmarkierungen in einem Bewegungsmodell angelernt sein und/oder in einer Karte gespeichert sein.

Gemäß einer weiteren Ausführungsform der Erfindung umfasst eine der aufgabenspezifischen Zonen einen Sperrbereich. Ein Sperrbereich stellt eine Zone dar, die von keiner mobilen Medizinkomponente befahren werden darf. Somit wird sichergestellt, dass keine mobile Medizinkomponente in den Sperrbereich fährt, in dem sich eventuell andere Objekte befinden (z.B. ein Patient), die durch das Einfahren einer mobilen Medizinkomponente gestört oder verletzt oder funktionsunfähig gemacht werden würden.

Gemäß einer weiteren Ausführungsform der Erfindung umfasst eine der aufgabenspezifischen Zonen einen Transferbereich. Der Transferbereich stellt eine Zone dar, über welche die Bewegung und/oder der Transfer einer mobilen Medizinkomponente zwischen dem Autonomiebereich und dem Bewegungsbereich oder zwischen dem Wartebereich und dem Bewegungsbereich erfolgt. Somit wird sichergestellt, dass keine mobile Medizinkomponente ohne Freigabesignal in den Bewegungsbereich fährt und eine Kollision verursacht.

Gemäß einer weiteren Ausführungsform der Erfindung umfasst eine der aufgabenspezifischen Zonen einen Bewegungsbereich. Der Bewegungsbereich stellt eine Zone dar, in dem sich eine mobile Medizinkomponente nach dem Empfang eines Freigabesignals bewegen darf. Über das Freigabesignal wird sichergestellt, dass keine mobile Medizinkomponente ohne Genehmigung in diese Zone einfährt. Eine Gefährdung von Personen in diesem Bereich wird somit minimiert. In dem Bewegungsbereich kann beispielsweise die Untersuchung und/oder Behandlung durchgeführt werden.

Gemäß einer weiteren Ausführungsform der Erfindung umfasst eine der aufgabenspezifischen Zonen einen Wartebereich. Der Wartebereich stellt eine Zone dar, in dem eine mobile Medizinkomponente in einem Wartezustand positioniert werden kann. Somit können Kollisionen zwischen einer wartenden mobilen Medizinkomponente und einer beweglichen Medizinkomponente vermieden werden.

Gemäß einer weiteren Ausführungsform der Erfindung umfasst eine der aufgabenspezifischen Zonen einen Autonomiebereich. Der Autonomiebereich stellt eine Zone dar, in dem sich die mobilen Medizinkomponenten autonom und ohne Freigabesignal bewegen können.

Gemäß einer weiteren Ausführungsform der Erfindung wird das Bewegen einer mobilen Medizinkomponente in den Bewegungsbereich über ein Freigabesignal freigegeben. Das Freigabesignal kann über eine Benutzerschnittstelle bereitgestellt werden. Die Benutzerschnittstelle ist ausgebildet eine Eingabe von einem Nutzer zu empfangen. Die Benutzerschnittstelle kann über ein elektronisches Gerät, beispielsweise einen Computer oder ein Handheld ausgebildet sein. Dies ermöglicht eine effiziente und schnelle Anwahl der entsprechenden mobilen Medizinkomponente. Zudem kann der Status oder die Statusnachricht der angewählten mobilen Medizinkomponente ausgegeben werden. Die Benutzerschnittstelle kommuniziert über die Kommunikationsverbindung mit den mobilen Medizinkomponenten. In einer alternativen Ausführungsform kann das Freigabesignal über ein Schaltpanel mit Schaltern und/oder Tastern bereitgestellt werden. Das Schaltpanel kann an einen Computer und/oder eine Steuerung zur Erzeugung des Freigabesignals angeschlossen sein. Der Computer und/oder die Steuerung kommunizieren über die Kommunikationsverbindung mit den mobilen Medizinkomponenten.

Gemäß einer weiteren Ausführungsform der Erfindung bewegen sich die mobilen Medizinkomponenten in der medizinischen Einrichtung über ein angelerntes Bewegungsmodell autonom. Dies hat den Vorteil, dass sich die mobilen Medizinkomponenten nach dem Empfang eines Anforderungssignals autonom zu der Einrichtungseinheit navigieren, in der das Anforderungssignal ausgelöst wurde. Das Anforderungssignal enthält die Informationen über die angeforderte mobile Medizinkomponente und in welcher Einrichtungseinheit die Verwendung der angeforderten mobilen Medizinkomponente erfolgen soll. Das Anforderungssignal kann über die Benutzerschnittstelle zum Erteilen des Freigabesignals bereitgestellt werden.

Gemäß einer weiteren Ausführungsform der Erfindung wird das Bewegungsmodell über ein manuell gefahrenes Bewegungsprofil angelernt. Das Bewegungsprofil kann für jede der verschiedenen Routen der jeweiligen mobilen Medizinkomponente durch ein manuelles Abfahren der Routen mit der jeweiligen Medizinkomponente erstellt werden. Die jeweiligen manuell gefahrenen Bewegungsprofile werden in dem Bewegungsmodel angelernt. In vorteilhafter Weise ist keine zusätzliche Hardware für die Lokalisierung notwendig.

Gemäß einer weiteren Ausführungsform der Erfindung wird die Bewegung über ein vorgegebenes Bewegungsprofil angelernt. In vorteilhafter Weise kann die Route direkt über ein Bewegungsprofil angelernt werden, wodurch kein manuelles Abfahren erfolgen muss. Das Bewegungsprofil umfasst Navigationsanweisungen zur Steuerung und/oder Navigation der mobilen Medizinkomponente in der medizinischen Einrichtung. Beispielsweise kann das Bewegungsprofil Navigationsanweisungen über die Bewegungsrichtung, Bewegungsgeschwindigkeit und/oder Bewegungsrichtungsänderung einer mobilen Medizinkomponente aufweisen.

Gemäß einer weiteren Ausführungsform der Erfindung orientieren und bewegen sich die mobilen Medizinkomponenten in der medizinischen Einrichtung über ein Lokalisierungssystem autonom. Das Lokalisierungssystem umfasst bekannte Koordinaten der medizinischen Einrichtung. Die Koordinaten beschreiben Punkte in den verschiedenen Einrichtungseinheiten in der medizinischen Einrichtung. Die Koordinaten können verschiedene Routen ausbilden. Entsprechend der mobilen Medizinkomponente und deren Verwendungsmöglichkeiten ergeben sich unterschiedliche Routen mit unterschiedlichen Koordinaten. Die Koordinaten können über ein Indoor-Positionsbestimmungsverfahren ermittelt werden. Die Indoor-Positionsbestimmung kann über WLAN, BLE, Ultra-wideband (UWB) und/oder Radio-Frequency Identification (RFID) erfolgen. Ein bestehendes WLAN-Netzwerk kann für ein WLAN-Positionierungssystem (WPS) genutzt werden. Das WPS ist ein Geolokalisierungssystem, das die Eigenschaften der nahegelegenen WLAN-Zugangspunkte und anderer drahtloser Zugangspunkte nutzt, um herauszufinden, wo sich die mobile Medizinkomponente befindet. Eine gängige und weit verbreitete Lokalisierungstechnik, die zur Positionierung mit drahtlosen Zugangspunkten verwendet wird, basiert auf der Messung der Intensität der empfangenen Signale. Typische Parameter, die für die Geolokalisierung der drahtlosen Zugangspunkte nützlich sind, sind SSID und MAC-Adresse.

Zur Bestimmung der Position über Blue Tooth Low Energie können Bluetooth-Signale von in der medizinischen Einrichtung installierten Bluetooth-Beacons empfangen werden. Die Position kann über eine Signalstärkemessung bestimmt werden.

Die mobile Abtastvorrichtung kann Bluetooth-Signale von Barken empfangen, die in der Scanumgebung installiert sind. Die Signalstärkemessung kann verwendet werden, um die Position den Barken zu bestimmen.

UWB ist eine Kurzstrecken-Funktechnik. Die Genauigkeit liegt bei unter 30 Zentimetern und ist damit deutlich höher als die von Beacons oder WLAN.

RFID umfasst ein automatisches und berührungsloses Verfahren zum Identifizieren und Lokalisieren mit Radiowellen.

Gemäß einer weiteren Ausführungsform der Erfindung bewegen sich die mobilen Medizinkomponenten in der medizinischen Einrichtung über ein SLAM-Verfahren autonom. Das SLAM-Verfahren löst rechnerisch das Problem, eine Karte (digitale Umgebungsdarstellung) einer unbekannten Umgebung (medizinischer Einrichtung) zu erstellen oder zu aktualisieren und gleichzeitig den Standort der mobilen Medizinkomponente in der Karte zu verfolgen. Die Karte wird inkrementell aufgebaut. Ausgehend von der Position der mobilen Medizinkomponente als Ausgangspunkt in der Karte kann eine Umgebungsdarstellung aufgebaut und kontinuierlich fortgesetzt werden. So gewinnt die mobile Medizinkomponente während der Scanzeit (Vermessung der Umgebung) immer besseres Wissen über die Umgebung und die Einrichtungseinheiten in der medizinischen Einrichtung. Dieses Wissen wird bei jeder Bewegungsaufgabe inkrementell verbessert. Die so erfassten Daten werden genutzt, um Steuerbefehle für das Antriebs- und Lenksystem der autonomen mobilen Medizinkomponente bereitzustellen, welches die autonome mobile Medizinkomponente entlang einer anwendungsfallspezifischen Scanroute steuert.

Gemäß einem zweiten Aspekt betrifft die Erfindung eine mobile Medizinkomponente aus einer Menge an mobilen Medizinkomponenten zur kooperativen Bewegungsüberwachung und/oder Bewegungssteuerung der Menge von mobilen Medizinkomponenten. Die mobile Medizinkomponente umfasst eine Antriebseinheit, die zum Antrieb der mobilen Medizinkomponente ausgebildet ist. Die Antriebseinheit umfasst einen Gleichstrommotor und kann ein Übersetzungsgetriebe umfassen.

Weiterhin umfasst die mobile Medizinkomponente eine Spannungsversorgung, die zur elektrischen Versorgung der einzelnen Komponenten der mobilen Medizinkomponente ausgebildet ist. Die Spannungsversorgung kann über eine Gleichspannungsquelle oder über eine Wechselspannungsquelle in Verbindung mit einem On-Board-Ladegerät in der mobilen Medizinkomponente oder einem Gleichrichter in der Wechselspannungsquelle geladen werden.

Die mobile Medizinkomponente umfasst eine Kommunikationsschnittstelle, die zum Aufbau einer Kommunikationsverbindung mit weiteren mobilen Medizinkomponenten in einer medizinischen Einrichtung ausgebildet ist. Die Kommunikationsschnittstelle ist ausgebildet, eine Kommunikationsverbindung über WLAN, Bluetooth, Bluetooth Low Energy oder Infrarot aufzubauen.

Weiterhin umfasst die mobile Medizinkomponente eine Sensoreinheit zur Erfassung der Umgebung der mobilen Medizinkomponente. In einer möglichen Ausführungsform umfasst die Sensoreinheit mindestens ein Lidarsystem oder ein Ladarsystem. Ein Lidar- (Lichtdetektion und -entfernung) bzw. Ladar- (Laserdetektion und -entfernung) System ist ein Vermessungsverfahren, das die Entfernung zum Ziel misst, indem es das Ziel mit gepulstem Licht, z.B. Laserlicht, beleuchtet und die reflektierten Impulse mit einem Sensor misst. Unterschiede in den Laser-Rücklaufzeiten und/oder der Wellenlänge können zudem genutzt werden, um die digitalen 3D-Darstellungen des Ziels zu erzeugen.

In einer weiteren möglichen Ausführungsform besteht die Sensoreinheit aus mindestens einem Kamerasystem. Die Kamera kann für Entfernungsmessungen, zur Unterscheidung und Identifikation von Objekten in der Umgebung und zur Bereitstellung von Daten für die Lokalisierung der mobilen Medizinkomponente verwendet werden.

In einer weiteren möglichen Ausführungsform besteht die Sensoreinheit aus mindestens einem Radarsystem. Das Radarsystem kann für Entfernungsmessungen verwendet werden.

In einer weiteren möglichen Ausführungsform beinhaltet die Sensoreinheit mindestens ein akustisches System. Akustische Systeme, wie z.B. Ultraschallsensoren, können zur Entfernungsmessung eingesetzt werden und um Daten für die Lokalisierung bereitzustellen.

In einer weiteren möglichen Ausführungsform besteht die Sensoreinheit aus mindestens einem Infrarot-System. Ein Infrarotsystem kann zur Entfernungsmessung eingesetzt werden und um Daten für die Lokalisierung bereitzustellen.

Die oben vorgestellten Systeme zur Abstandsmessung können einzeln oder in Kombination verwendet werden, um die Scanumgebung (also die Umgebung, in der sich die mobile Medizinkomponente bewegen soll) zu scannen und/oder Objekte oder weitere mobile Medizinkomponenten in der Scanumgebung zu erkennen.

Weiterhin umfasst die mobile Medizinkomponente eine Speichereinheit zur Speicherung des angelernten Bewegungsmodells und/oder von Karten der medizinischen Einrichtung. Die Speichereinheit kann einen Nur-Lese-Speicher (ROM) und/oder einen Direktzugriffsspeicher (RAM) beinhalten. Die Speichereinheit kann auch ein Festplattenlaufwerk zum Lesen von und Schreiben auf eine Festplatte beinhalten. Die Speichermedien bieten eine nichtflüchtige Speicherung von maschinenlesbaren Anweisungen, Datenstrukturen, Programmmodulen, des Bewegungsmodells und weiteren Daten.

Zudem umfasst die mobile Medizinkomponente eine Prozessoreinheit (wenigstens einen Prozessor) zum kooperativen Überwachen und/oder Steuern der Bewegung jeder mobilen Medizinkomponente in der medizinischen Einrichtung und/oder in den Einrichtungseinheiten durch das Bereitstellen von Steuersignalen zum Ansteuern der Antriebseinheit. Die Prozessoreinheit kann auch in einem integrierten Schaltkreis ausgebildet werden.

Gemäß einem dritten Aspekt betrifft die Erfindung ein Verfahren zum Betreiben eines Systems wie vorstehend beschrieben durch Bereitstellen von lokal und autonom auf der medizinischen Komponente erzeugten Steuersignalen zur kooperativen Bewegungsüberwachung und/oder Bewegungssteuerung der Menge an mobilen Medizinkomponenten in einer Einrichtung und/oder in einer Vielzahl an Einrichtungseinheiten der Einrichtung.

Gemäß einem vierten Aspekt betrifft die Erfindung ein Verfahren zum Betreiben einer mobilen Medizinkomponente einer Menge an mobilen Medizinkomponenten in einer medizinischen Einrichtung umfassend eine Antriebseinheit, eine Spannungsversorgung, eine Kommunikationsschnittstelle, eine Sensoreinheit, eine Speichereinheit und eine Prozessoreinheit. Das Verfahren umfasst die folgenden Schritte:
- Aktivieren der Kommunikationsschnittstelle zum kontinuierlichen Empfangen von Navigationssignalen von jeder der mobilen Medizinkomponenten der Menge an mobilen Medizinkomponenten in der medizinischen Einrichtung;
- Empfangen von Navigationssignalen von jeder der mobilen Medizinkomponenten;
- Erzeugen von Steuersignalen für die Antriebseinheit zur Bewegung der mobilen Medizinkomponente auf Basis der empfangenen Navigationssignale und/oder eines Bewegungsmodells und/oder eines Anforderungssignals;
- Erzeugen von Navigationssignalen über ein Kommunikationsprotokoll auf Basis der erzeugten Steuersignale; und
- Senden der erzeugte Navigationssignale über die Kommunikationsschnittstelle zur kooperativen Überwachung und/oder Steuerung jeder mobilen Medizinkomponente der Vielzahl an mobilen Medizinkomponenten in der medizinischen Einrichtung und/oder in den Einrichtungseinheiten.

In einer Ausführungsform der mobilen Medizinkomponente kann diese eine Benutzerschnittstelle zur Kommunikation mit einem User umfassen. Die Benutzerschnittstelle ist ausgebildet visuelle und/oder akustische Informationen bereitzustellen. Zudem ist die Benutzerstelle ausgebildet Anforderungseingaben von einem Nutzer zu empfangen.

Die vorstehend beschriebenen, erfindungsgemäßen Ausführungsform des Verfahrens gemäß dem dritten Aspekt der Erfindung kann auch als Computerprogramm ausgebildet sein, wobei ein Computer zur Durchführung des oben beschriebenen, erfindungsgemäßen Verfahrens veranlasst wird, wenn das Computerprogramm auf dem Computer bzw. auf einem Prozessor des Computers ausgeführt wird. Das Computerprogramm kann als Signal per Download bereitgestellt oder in einer Speichereinheit des Computers oder der mobilen Medizinkomponente mit darin enthaltenem computerlesbarem Programmcode gespeichert werden, um die mobile Medizinkomponente zur Ausführung von Anweisungen gemäß dem oben genannten Verfahren zu veranlassen. Dabei kann das Computerprogramm auch auf einem maschinenlesbaren Speichermedium gespeichert sein. Eine alternative Aufgabenlösung sieht ein Speichermedium vor, das zur Speicherung des vorstehend beschriebenen, computer-implementierten Verfahrens bestimmt ist und von einem Computer oder Prozessor lesbar ist.

Die obigen Ausgestaltungen und Weiterbildungen lassen sich, sofern sinnvoll, beliebig miteinander kombinieren. Weitere mögliche Ausgestaltungen, Weiterbildungen und Implementierungen der Erfindung umfassen auch nicht explizit genannte Kombinationen von zuvor oder im Folgenden bezüglich der Ausführungsbeispiele beschriebenen Merkmalen der Erfindung. Insbesondere wird dabei der Fachmann auch Einzelaspekte als Verbesserung oder Ergänzungen zu der jeweiligen Grundform der vorliegenden Erfindung hinzufügen.

Die vorliegende Erfindung wird nachfolgend anhand der in den schematischen Figuren der Zeichnungen angegebenen Ausführungsbeispiele näher erläutert. Es zeigen dabei:
- Fig. 1: eine schematische Darstellung einer Ausführungsform eines Systems zur kooperativen Bewegungsüberwachung und/oder Bewegungssteuerung;
- Fig. 2: ein Blockdiagram eines Kommunikationsschichten-Modells zur Kommunikation der mobilen Medizinkomponenten;
- Fig. 3: eine schematische Darstellung eines zeitlichen Ablaufs der Registrierung einer neuen mobilen Medizinkomponente;
- Fig. 4: eine schematische Darstellung eines zeitlichen Ablaufs der Datenkommunikation zwischen den mobilen Medizinkomponenten;
- Fig. 5: eine schematische Darstellung eines zeitlichen Ablaufs der Bewegungsanforderung einer mobilen Medizinkomponente;
- Fig. 6: eine schematische Darstellung eines zeitlichen Ablaufs einer Master-Anforderung einer mobilen Medizinkomponente;
- Fig. 7: ein weiteres Blockdiagramm gemäß einer Ausführungsform der mobilen Medizinkomponenten;
- Fig. 8: ein Ablaufdiagramm gemäß einer Ausführungsform des erfindungsgemäßen Verfahrens.

Die beiliegenden Zeichnungen sollen ein weiteres Verständnis der Ausführungsformen der Erfindung vermitteln. Sie veranschaulichen Ausführungsformen und dienen im Zusammenhang mit der Beschreibung der Erklärung von Prinzipien und Konzepten der Erfindung. Andere Ausführungsformen und viele der genannten Vorteile ergeben sich im Hinblick auf die Zeichnungen. Die Elemente der Zeichnungen sind nicht notwendigerweise maßstabsgetreu zueinander gezeigt.

In den Figuren der Zeichnung sind gleiche, funktionsgleiche, und gleich wirkende Elemente, Merkmale und Komponenten - sofern nichts anderes ausgeführt ist - jeweils mit denselben Bezugszeichen zu versehen.

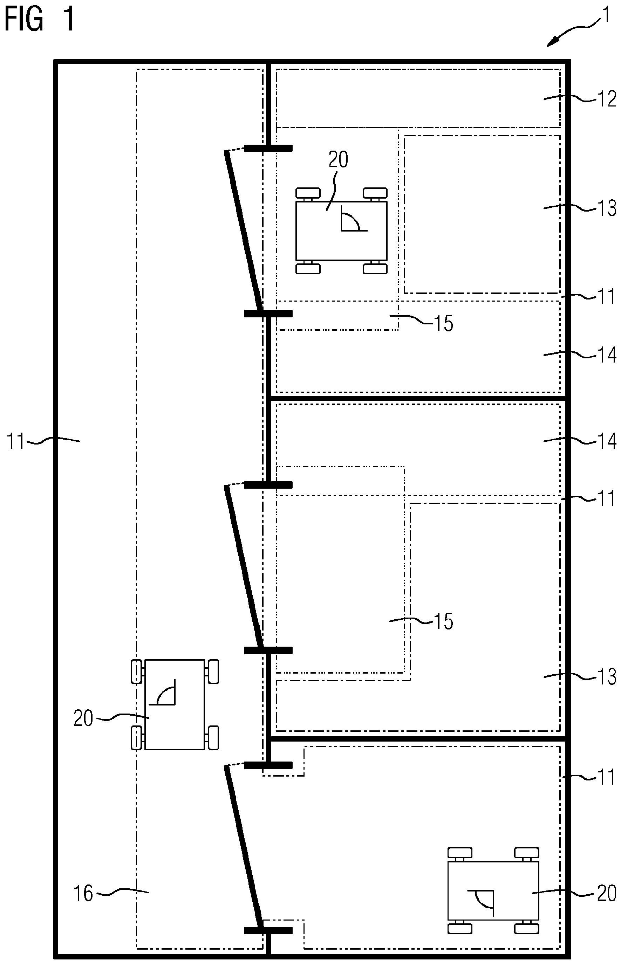

Fig. 1 zeigt eine schematische Darstellung einer Ausführungsform eines Systems zur kooperativen Bewegungsüberwachung und/oder Bewegungssteuerung. Das System ist in einer medizinische Einrichtung 1 implementiert. Die medizinische Einrichtung 1 umfasst beispielsweise ein Krankenhaus, eine Ambulanz, ein medizinisches Forschungslabor, eine Krankenstation oder eine Poliklinik. Die medizinische Einrichtung 1 kann in eine Vielzahl an Einrichtungseinheiten 11 aufgeteilt sein. Eine Einrichtungseinheit 11 kann beispielsweise einen Raum, einen Korridor, eine Treppe und/oder einen Aufzug beinhalten. Die Einrichtungseinheit 11 kann in eine Vielzahl von aufgabenspezifischen Zonen (12, 13, 14, 15, 16) unterteilt werden. Die aufgabenspezifische Zone kann als ein Sperrbereich 12 ausgebildet sein. Der Sperrbereich 12 darf durch keine mobile Medizinkomponente 20 befahren werden.

In der medizinischen Vorrichtung 1 kann eine Vielzahl an mobilen Medizinkomponenten 20 eingesetzt werden. Die Vielzahl der mobilen Medizinkomponenten 20 stehen über eine Kommunikationsverbindung zueinander in Verbindung und kommunizieren über diese Verbindung kontinuierlich, um über ein Kommunikationsprotokoll kooperativ die Bewegung jeder mobilen Medizinkomponente 20 der Vielzahl an mobilen Medizinkomponenten 20 in der medizinischen Einrichtung 1 und/oder den Einrichtungseinheiten 11 zu überwachen und/oder zu steuern. Eine mobile Medizinkomponente 20 kann ein Untersuchungsgerät für ein bildgebendes Verfahren, beispielsweise CT, MRT, Röntgen oder Ultraschall umfassen. Weiterhin umfassen medizinische Komponenten 20 auch die Komponenten, welche zur Heilung und/oder Lebenserhaltung eingesetzt werden.

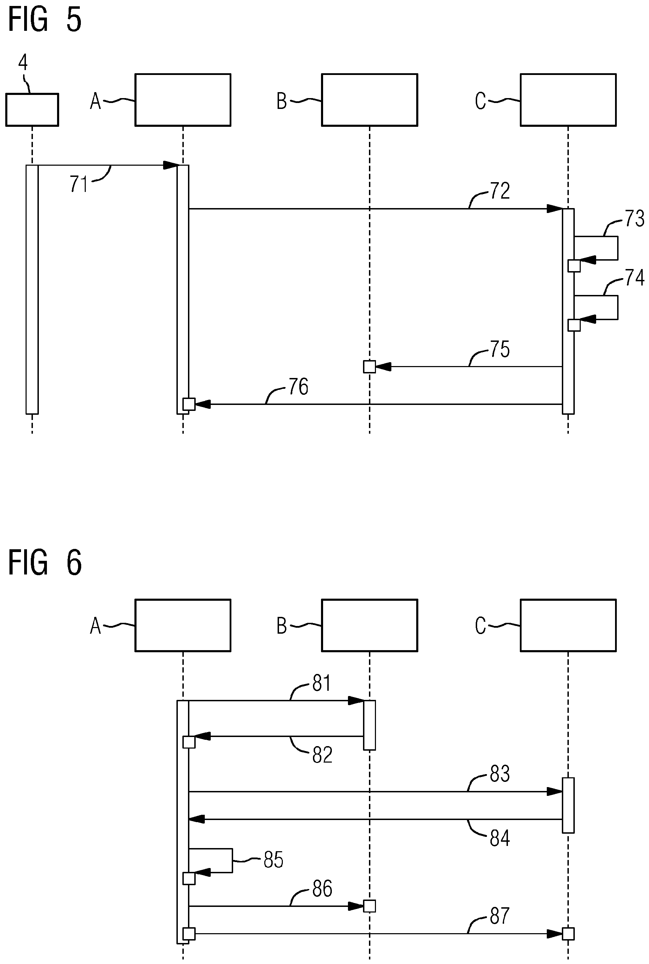

Die aufgabenspezifische Zone kann weiterhin als ein Bewegungsbereich 13 ausgebildet sein. Der Bewegungsbereich 13 kann durch eine mobile Medizinkomponente 20 nach dem Empfang eines Freigabesignals befahren werden. Das Freigabesignal kann durch einen Nutzer über eine Benutzerschnittstelle 4 (vgl. Fig. 5) bereitgestellt werden. Zudem dürfen sich die mobilen Medizinkomponenten 20 in dem Bewegungsbereich 13 nur bewegen, wenn ein Freigabesignal zur Bewegung über die Benutzerschnittstelle 4 bereitstellt wurde. Somit kann die Sicherheit für die Nutzer in dem Bewegungsbereich 13 erhöht werden. In einer Ausführungsform werden auf die mobilen Medizinkomponenten 20, die im Bewegungsbereich 13 positioniert sind, ein Master-Slave-Prinzip für die Kommunikation und Steuerung angewendet. Hierbei ist eine mobile Medizinkomponente 20 als Master angewählt und die weiteren mobilen Medizinkomponenten 20 sind als Slave angewählt. Die mobile Medizinkomponente 20 mit der Masterfunktion kann die weiteren mobilen Medizinkomponenten 20 im Bewegungsbereich 13 automatisiert fernsteuern. Die Masterfunktion einer mobilen Medizinkomponente 20 kann auch an andere mobile Medizinkomponenten 20 übertragen werden. Das Übertragen kann durch eine Nutzerfreigabe über die Benutzerschnittstelle 4 erfolgen. Die mobilen Medizinkomponenten 20 können auch manuell durch einen Nutzer in der medizinischen Einrichtung 1 bewegt werden.

Wenn eine mobile Medizinkomponente 20 eine Zielposition in dem Bewegungsbereich 13 nicht anfahren kann, weil beispielsweise eine andere mobile Medizinkomponente 20 die Zielposition belegt, erfolgt eine Kommunikation zwischen den beteiligten mobilen Medizinkomponenten 20. Die mobile Medizinkomponente 20 meldet, dass diese die Zielposition anfahren möchte. Die mobile Medizinkomponente 20, welche die Zielposition belegt, kann entscheiden, ob sie den Bewegungsbereich 13 und somit die Zielposition verlassen kann, indem es beispielsweise in den Wartebereich 14 fährt. In einer Ausführungsform kann die blockierte mobile Medizinkomponente 20 einem Nutzer über eine Benutzerschnittstelle anzeigen, dass ein Konflikt vorliegt. Der Nutzer kann in diesem Fall eine Konfliktlösung bereitstellen.

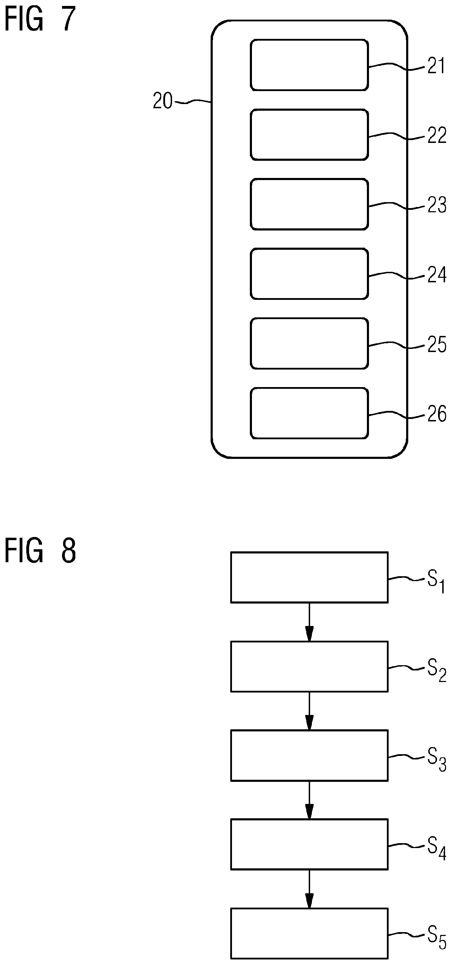

Die aufgabenspezifische Zone kann weiterhin als ein Wartebereich 14 ausgebildet sein. In dem Wartebereich 14 können mobile Medizingeräte 20 abgestellt werden, die nicht in Verwendung sind. Die mobilen Medizingeräte 20 können automatisch in dem Wartebereich 14 abgestellt werden, wenn diese nicht mehr benötigt werden. Hierfür wird die mobile Medizinkomponente 20 in den Wartebereich 14 bewegt. Es kann ein orthogonales Verlassen des Bewegungsbereiches 13 in den Wartebereich 14 erfolgen. Ein orthogonales Verlassen kann beispielsweise so erfolgen, dass auf Freigabe durch einen Nutzer, die mobile Medizinkomponente 20 von dem Bewegungsbereich 13 in den Transferbereich 15 bewegt wird und aus dem Transferbereich 15 autonom in den Wartebereich 14 verfährt. Der Transferbereich 15 stellt hierbei einen Bereich dar, über den der Transfer zwischen dem Autonomiebereich 16 und dem Bewegungsbereich 13, sowie der Transfer zwischen dem Bewegungsbereich 13 und dem Wartbereich 15 gesteuert wird. Der Wartebereich 15 kann eine Vielzahl an Wartepositionen umfassen. Die Wartepositionen werden automatisch gefunden. In einer Ausführungsform werden hierzu durch eine mobile Medizinkomponente 20, die Positionen der weiteren mobilen Medizinkomponenten 20 abgefragt. Hierdurch wird ermittelt, welche Warteposition in dem Wartbereich 15 noch frei ist und verwendet werden kann. In einer weiteren Ausführungsposition kann eine freie Warteposition über die Sensoreinheit 24 (vgl. Fig. 7) der mobilen Medizinkomponente 20 ermittelt werden.

Bei einer Bewegung von mehreren mobilen Medizinkomponenten 20 hat die Bewegung der mobilen Medizinkomponenten 20 aus dem Bewegungsbereich 13 in den Wartebereich 15 Vorrang. Erst wenn die mobilen Medizinkomponenten 20, welche aus dem Bewegungsbereich 13 in den Wartebereich 15 auf eine Warteposition fahren den Bewegungsbereich 13 vollständig verlassen haben, erfolgt die Bewegung einer anderen mobilen Medizinkomponente 20 in den Bewegungsbereich 13. Somit wird die gleichzeitige Ein- und Ausfahrbewegung mehrerer mobiler Medizinkomponenten 20 vermieden. Das Kollisionsrisiko wird reduziert.

In einer vorteilhaften Ausführungsform bleiben die mobilen Medizinkomponenten 20, welche sich auf einem Kollisionskurs bewegen, stehen. Die mobilen Medizinkomponenten 20 werden angehalten bevor ein Kollisionsfall eintritt. Der Stopp kann einem Nutzer über ein akustisches und/oder visuelles Signal signalisiert werden. Beispielsweise kann das akustische und/oder visuelle Signal über eine Benutzerschnittstelle der mobilen Medizinkomponente 20 bereitgestellt werden. Der Stopp kann als Stoppsignal auch an alle oder ausgewählte andere mobile Medizinkomponenten übertragen werden.

Der Autonomiebereich 16 stellt eine Zone dar, in dem sich die mobile Medizinkomponente 20 autonom, ohne Benutzervorgaben bewegt. Der Autonomiebereich 16 umfasst die Flure, Gänge und Korridore, sowie Lager zum Abstellen der mobilen Medizinkomponenten 20. Wird eine neue mobile Medizinkomponente 20 in einem bestimmten Bewegungsbereich 13 einer Einrichtungseinheit 11 benötigt, kann diese über ein Anforderungssignal angefordert werden. Das Anforderungssignal kann über die Benutzerschnittstelle 4 bereitgestellt werden. Die angeforderte mobile Medizinkomponente 20 bewegt sich dann autonom, beispielsweise aus einem Lager in den Autonomiebereich 16. Über den Autonomiebereich 16 kann sich die mobile Medizinkomponente 20 selbstständig bis in den Transferbereich 15 der jeweiligen Einrichtungseinheit 11 bewegen. Das Erreichen des Transferbereiches 15 der jeweiligen Einrichtungseinheit 11 wird einem Nutzer signalisiert, beispielsweise über eine Anzeige in der Benutzerschnittstelle 4. Über die Benutzerschnittstelle 4 muss aktiv ein Freigabesignal bereitgestellt werden, bevor die angeforderte mobile Medizinkomponente 20 in den Bewegungsbereich 13 der Einrichtungseinheit 11 einfahren kann. In einer bevorzugten Ausführungsform meldet sich die angeforderte mobile Medizinkomponente 20 auch an der gerade aktiven Masterkomponente an. In einer Ausführungsform ist der Autonomiebereich 16 so ausgestaltet, dass für jede Fahrrichtung eine Fahrspur bereitgestellt wird. In einer weiteren Ausführungsform sind die Fahrspuren mit einer autonomen Spurführung realisiert. Die Spurführung kann auch über ein vorgegebenes Bewegungsprofil angelernt werden.

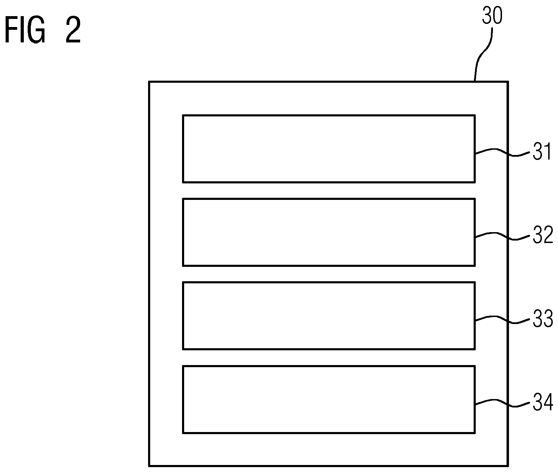

Fig. 2 zeigt ein Blockdiagram eines Kommunikationsschichten-Modells 30 zur Kommunikation der mobilen Medizinkomponenten 20.

Die Schicht 31 umfasst das kooperative Kommunikationsprotokoll, beispielsweise ein Bewegungsplaner-Protokoll gemäß der vorliegenden Erfindung.

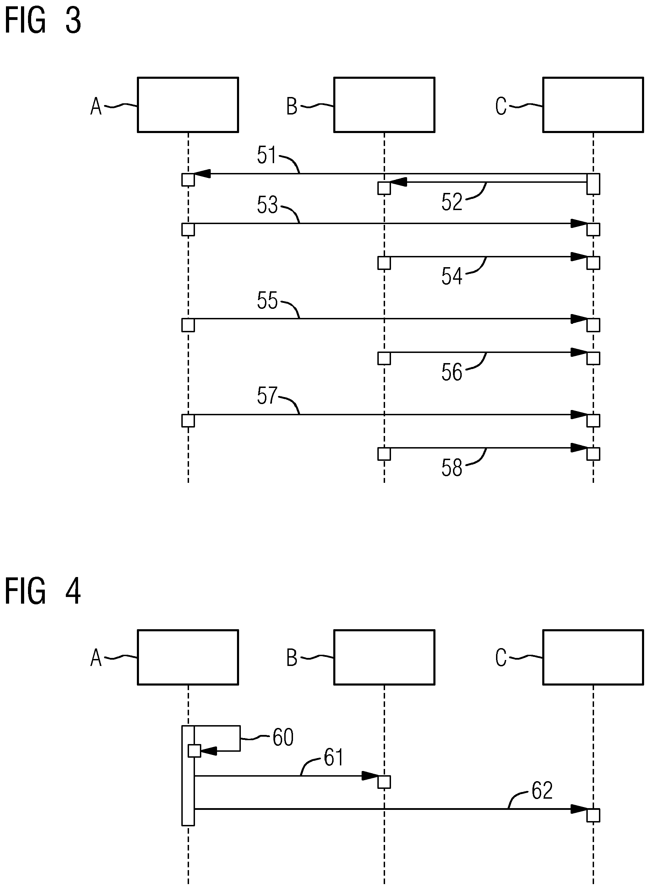

Durch das kooperative Bewegungsplaner-Protokoll werden die folgenden Nachrichten definiert:
/robot_broadcast 51 (vgl. Fig. 3) umfasst eine Broadcast Nachricht zur Anmeldung einer neuen mobile Medizinkomponente 20;
/robot _status 53, 54, 75, 76 (vgl. Fig. 3) umfasst eine Statusnachricht, welche den aktuellen Zustand der mobilen Medizinkomponente 20 sendet, wie beispielsweise den Namen, den Zustand, ob die mobile Medizinkomponente 20 in der Master- oder Slave-Funktion ist;
/map_update 55, 56, 61, 62 (vgl. Fig. 3) umfasst das Senden eines Updates der Karte an alle mobilen Medizinkomponenten 20; Es kann ein Belegtheitsgitter ähnlich wie bei dem Robot Operating System (ROS) verwendet werden, welches zellenbasiert den verfügbaren Raum zerlegt und für jede Zelle speichert, ob die Zelle belegt ist oder nicht;
/floor_update 57, 58, 86, 87 (vgl. Fig. 3) umfasst ein Update des Etagenplans mit den Bewegungsbereichen in den Einrichtungseinheiten 11; Zudem wir für jede Einrichtungseinheit 11 der Standardmaster mit Verbindungsdaten abgelegt;
/move_request 72 (vgl. 72) umfasst die Anforderung einer Bewegung einer mobilen Medizinkomponente 20 auf eine Zielposition in der Karte gemäß den oben aufgestellten Regeln;
/master_request 81, 83 (vgl. Fig. 6) umfasst die Anforderung der Übernahme der Masterfunktion von einer anderen mobilen Medizinkomponente 20.

Die Schicht 32 umfasst das Robot Operating Messaging System (ROS). Über das Robot Operating Messaging System kann in vorteilhafter Weise eine verteilte Kommunikation über mehrere mobile Medizinkomponenten 20 ohne einen dedizierten Kommunikationsmaster erfolgen.

Die Schicht 33 umfasst die Transportschicht. In der Transportschicht erfolgt die Segmentierung des Datenstroms und die Stauvermeidung. Über die Transportschicht wird ein einheitlicher Zugriff der anwendungsorientierten Schichten 31, 32 bereitgestellt, so dass die Eigenschaften des Kommunikationsnetzes nicht zu berücksichtigen sind. Als Protokolle können TCP/IP (Transmission Control Protocol/Internet Protocol), UDP (User Datagramm Protocol), SCTP (Stream Control Transmission Protocol), DCCP (Datagramm Congestion Control Protocol) verwendet werden.

Die Schicht 34 umfasst die Verbindungssicherungsschicht, die eine zuverlässige und fehlerfreie Übertragung gewährleisten und den Zugriff auf das Übertragungsmedium bereitstellen soll, beispielsweise WLAN, Bluetooth, Bluetooth Low Energy.

Über das Kommunikationsprotokoll können eine beliebige Anzahl an mobiler Medizinkomponenten 20 hinzugefügt, überwacht und/oder gesteuert werden. Zudem wird kein zentraler Planungs- und/oder Kooperationsrechner benötigt. Die Überwachung und/oder Steuerung erfolgt dezentral auf den jeweiligen mobilen Medizinkomponenten 20.

Fig. 3 zeigt eine schematische Darstellung eines zeitlichen Ablaufs der Registrierung einer neuen mobilen Medizinkomponente 20. In Fig. 3 bezeichnen die Bezugszeichen A, B und C eine erste, zweite und dritte mobile Medizinkomponente 20. In Fig. 3 ist die Registrierung einer neuen mobilen Medizinkomponente C dargestellt. Die neue mobile Medizinkomponente C sendet eine /robot_broadcast Nachricht 51, 52 an die verfügbaren mobilen Medizinkomponenten A, B und macht sich bekannt. Die verfügbaren Medizinkomponenten A, B senden als Antwort auf die /robot_broadcast - Nachricht 51, 52 ihren Status über die Nachricht /robot_status 53, 54 an die neue mobile Medizinkomponente C. Die verfügbaren Medizinkomponenten A, B senden zudem eine Karte und einen Plan über die Einrichtungseinheiten 11 über die Nachricht /map_update 55, 56 und über die Nachricht /floor_update 57, 58. Die neue mobile Medizinkomponente C kann sich aus den Daten eine Karte und einen Plan über die Einrichtungseinheiten 11 erzeugen.

Fig. 4 zeigt eine schematische Darstellung eines zeitlichen Ablaufs der Datenkommunikation zwischen den mobilen Medizinkomponenten A, B, C. Die Datenkommunikation kann beispielsweise Daten zur Aktualisierung einer Karte umfassen. In Fig. 4 hat die erste mobile Medizineinheit A über die Sensoreinheit 24 (vgl. Fig. 7) neue Informationen detektiert und aktualisiert die Karte 60 auf Basis der neuen Informationen. Die erste mobile Medizineinheit A sendet die Nachricht /map_update 61, 61 an die weiteren mobilen Medizineinheiten B, C. Die Karten der weiteren mobilen Medizineinheiten B, C werden aktualisiert.

Fig. 5 zeigt eine schematische Darstellung eines zeitlichen Ablaufs der Bewegungsanforderung einer mobilen Medizinkomponente A, B, C. In Fig. 5 wird beispielsweise eine neue mobile Medizinkomponente C aus einem Lager angefordert. Für die Anforderung wird ein Anforderungssignal über die Benutzerschnittstelle 4 bereitgestellt. Das Anforderungssignal kann die Informationen über die angeforderte mobile Medizinkomponente und den anfordernden Bewegungsbereich 13 in einer Einrichtungseinheit 11 umfassen. Die dritte mobile Medizinkomponente C empfängt eine Nachricht /move_request 72. Die dritte mobile Medizinkomponente C überprüft 73, ob der Transferbereich 15 der anfordernden Einrichtungseinheit 11 frei ist. Ergibt die Überprüfung 73, dass der Transferbereich 15 der anfordernden Einrichtungseinheit 11 frei ist, wird eine autonome Bewegung der dritten mobile Medizinkomponente C gestartet 74. Während der Bewegung übermittelt die dritte mobile Medizinkomponente C ihren Status, beispielsweise die Position, durch die Nachricht /robot_status 75, 76 an die weiteren mobilen Medizingeräte A, B. Dies ist dann der Fall, wenn die weiteren mobilen Medizingeräte A, B in der anfordernden Einrichtungseinheit 11 (vorhanden) sind und die Benutzerschnittstelle der ersten mobilen Medizinkomponente A für die Anforderung der dritten mobilen Medizinkomponente C verwendet wurde.

Fig. 6 zeigt eine schematische Darstellung eines zeitlichen Ablaufs einer Master-Anforderung einer mobilen Medizinkomponente 20. In dem in der Fig. 6 dargestellten Beispiel ist die zweite mobile Medizinkomponente B als Master gesetzt und die weiteren mobilen Medizinkomponenten A, C sind Slave. Die erste mobile Medizinkomponente A sendet eine Nachricht /master_request 81, 83 an die mobilen Medizinkomponenten B, C, um einen Wechsel auf ,Master' zu erwirken. Die mobilen Medizinkomponenten B, C bestätigen 82, 84 den Empfang des Request's und die zweite mobile Medizinkomponente B als aktueller Master quittiert die Masterfunktion. Die erste mobile Medizinkomponente A setzt 85 die Masterfunktion. Als Masterkomponente kann die erste mobile Medizinkomponente A ein Aktualisieren des Raumplans über die Nachricht /floor_update 86, 87 starten.

Fig. 7 zeigt ein weiteres Blockdiagramm gemäß einer Ausführungsform der mobilen Medizinkomponenten 20. Die mobile Medizinkomponente 20 aus einer Menge an mobilen Medizinkomponenten 20 zur kooperativen Bewegungsüberwachung und/oder Bewegungssteuerung der Menge von mobilen Medizinkomponenten 20 in einer medizinischen Einrichtung 1 umfasst eine Antriebseinheit 21. Die Antriebseinheit 21 ist zum Antrieb der mobilen Medizinkomponente 20 ausgebildet. Die Antriebseinheit ist als ein Gleichstrommotor ausgebildet. Weiterhin umfasst die mobile Medizinkomponente 20 eine Spannungsversorgung 22. Die Spannungsversorgung ist zur elektrischen Versorgung der einzelnen Komponenten der mobilen Medizinkomponente 20 ausgebildet. Die Spannungsversorgung 22 umfasst eine Gleichstromversorgung die über eine Gleichspannungsquelle oder einen Wechselspannungsquelle mit einem AC/DC Wandler geladen werden kann. Das Laden kann in vorteilhafter Weise während einer Wartepause in einem Wartebereich 15 oder in einem Lager erfolgen. Zudem umfasst die mobile Medizinkomponente 20 eine Kommunikationsschnittstelle 23. Die Kommunikationsschnittstelle 23 ist zum Aufbau einer Kommunikationsverbindung mit weiteren mobilen Medizinkomponenten 20 in einer medizinischen Einrichtung 1 ausgebildet. Die Kommunikationsschnittstelle ist ausgebildet eine WLAN-Verbindung, eine Bluetooth- oder Bluetooth low Energy-Verbindung aufzubauen. Weiterhin umfasst die mobile Medizinkomponente 20 eine Sensoreinheit 24 zur Erfassung der Umgebung der mobilen Medizinkomponente 20. Die Sensoreinheit 24 ist ausgebildet, um die Umgebung der mobilen Medizinkomponente 20 zu scannen und Informationen über die Entfernung und die Ausgestaltung von Objekten innerhalb der Umgebung bereitzustellen. Die Sensoreinheit 24 umfasst beispielsweise Lidar, Radar, Laser und/oder eine Kamera. Auf Basis der bereitgestellten Informationen kann eine Karte erzeugt werden. Die Karte kann an die weiteren mobilen Medizinkomponenten 20 übertragen werden. Zudem umfasst die mobile Medizinkomponente 20 eine Speichereinheit 25 zur Speicherung des angelernten Bewegungsmodells und/oder von Karten der medizinischen Einrichtung 1. Zudem umfasst die mobile Medizinkomponente 20 eine Prozessoreinheit 26 zum kooperativen Überwachen und/oder Steuern der Bewegung jeder mobilen Medizinkomponente 20 in der medizinischen Einrichtung 1 und/oder in den Einrichtungseinheiten 11 durch Bereitstellen von Steuersignalen zum Ansteuern der Antriebseinheit 21.

In einer bevorzugten Ausführungsform der Erfindung können die mobilen Medizinkomponenten 20 mit unterschiedlichen Ressourcen ausgebildet sein, so dass z.B. eine erste mobile Medizinkomponente 20 mit geringen Rechenressourcen und einen zweite mobile Medizinkomponente 20 mit im Vergleich relativ höheren Rechenressourcen ausgebildet ist. Dann kann die erste mobile Medizinkomponente 20 die zweite mobile Medizinkomponente 20 beauftragen, deren Berechnungen (z.B. zur Navigation, zum Aufbau der Karte und/oder zur Berechnung im Rahmen der Steuerung und Überwachung) kommissarisch bzw. stellvertretend zu übernehmen und das Ergebnis an die erste mobile Medizinkomponente 20 zurückzusenden. Damit kann die erste mobile Medizinkomponente 20 von Rechenlast befreit werden und dennoch am Verfahren teilnehmen.

In einer Ausführungsform umfasst die mobile Medizinkomponente 20 eine Benutzerschnittstelle. Die Benutzerschnittstelle kann eine Eingabeeinheit, beispielsweise ein Eingabepanel zum Empfangen von Nutzereingaben und eine Ausgabeeinheit, beispielsweise ein Display zur Ausgabe von Informationen umfassen. Weiterhin kann die Benutzerschnittstelle eine akustische Ausgabeeinheit, beispielsweise einen Lautsprecher umfassen. In einer alternativen Ausführungsform kann die Benutzerschnittstelle ganzheitlich in einem Touch-Display oder einem Handheld integriert sein und einen Lautsprecher zur Ausgabe akustischer Warntöne umfassen.

Fig 8 zeigt ein Ablaufdiagramm gemäß einer Ausführungsform des erfindungsgemäßen Verfahrens. Das Verfahren umfasst bei der dargestellten Ausführungsform mehrere Schritte. In einem ersten Schritt S1 wird die Kommunikationsschnittstelle 23 zum kontinuierlichen Empfangen von Navigationssignalen von jeder der mobilen Medizinkomponenten 20 der Menge an mobilen Medizinkomponenten 20 in der medizinischen Einrichtung 1 aktiviert. In einem weiteren Schritt S2 erfolgt ein Empfangen von Navigationssignalen von jeder der mobilen Medizinkomponenten 20. In einem weiteren Schritt S3 werden Steuersignale für die Antriebseinheit zur Bewegung der mobilen Medizinkomponente 20 auf Basis der empfangenen Navigationssignale und/oder eines Bewegungsmodells und/oder eines Anforderungssignals erzeugt. In einer Ausführungsform kann das Bewegungsmodell über ein manuell gefahrenes Bewegungsprofil angelernt werden. In einer weiteren Ausführungsform kann das Bewegungsmodell über ein vorgegebenes Bewegungsprofil angelernt werden. In einem weiteren Schritt S4 werden Navigationssignale über ein Kommunikationsprotokoll auf Basis der erzeugten Steuersignale erzeugt. Über das Kommunikationsprotokoll werden die Nachrichten zur Navigation der mobilen Medizinkomponenten 20 in der medizinischen Einrichtung 1 definiert. In einem weiteren Schritt S5 werden die erzeugten Navigationssignale über die Kommunikationsschnittstelle 23 zur kooperativen Überwachung und/oder Steuerung jeder mobilen Medizinkomponente 20 der Vielzahl an mobilen Medizinkomponenten 20 in der medizinischen Einrichtung 1 und/oder in den Einrichtungseinheiten 11 gesendet.

## Patentansprüche

1. System zur kooperativen Bewegungsüberwachung und/oder Bewegungssteuerung von mobilen Medizinkomponenten (20) in einer medizinischen Einrichtung (1) mit einer Vielzahl an Einrichtungseinheiten (11),
umfassend eine Vielzahl an mobilen Medizinkomponenten (20), die über eine Kommunikationsverbindung in Datenaustausch stehen und kontinuierlich kommunizieren, um über ein Kommunikationsprotokoll kooperativ die Bewegung jeder mobilen Medizinkomponente (20) der Vielzahl an mobilen Medizinkomponenten (20) in der medizinischen Einrichtung (1) und/oder in den Einrichtungseinheiten (11) zu überwachen und/oder zu steuern.

2. System nach Anspruch 1, wobei das Kommunikationsprotokoll der mobilen Medizinkomponenten (20) als Master-Slave-Protokoll ausgebildet ist, wobei eine mobile Medizinkomponente (20) als Master angewählt ist und die weiteren mobilen Medizinkomponenten (20) als Slave angewählt sind.

3. System nach einem der vorherigen Ansprüche, wobei eine Einrichtungseinheit (11) der Vielzahl an Einrichtungseinheiten (11) der medizinischen Einrichtung (1) wenigstens zwei getrennte aufgabenspezifische Zonen (12, 13, 14, 15, 16) umfasst.

4. System nach Anspruch 3, wobei eine der aufgabenspezifischen Zonen (12, 13, 14, 15, 16) einen Sperrbereich (12), einen Transferbereich (15), einen Bewegungsbereich (13), einen Wartebereich (14) oder einen Autonomiebereich (16) umfasst.

5. System nach Anspruch 4, wobei das Bewegen einer mobilen Medizinkomponente (20) in den Bewegungsbereich (13) über ein Freigabesignal freigegeben wird.

6. System nach einem der vorherigen Ansprüche, wobei sich die mobilen Medizinkomponenten (20) in der medizinischen Einrichtung (1) über ein angelerntes Bewegungsmodell der medizinischen Einrichtung (1) autonom bewegen.

7. System nach Anspruch 6, wobei das Bewegungsmodell über ein manuell gefahrenes Bewegungsprofil angelernt wird.

8. System nach Anspruch 6, wobei die Bewegung über ein vorgegebenes Bewegungsprofil angelernt wird.

9. System nach einem der vorherigen Ansprüche 1 bis 5, wobei sich die mobilen Medizinkomponenten (20) in der medizinischen Einrichtung (1) über ein Lokalisierungssystem orientieren und autonom bewegen.

10. System nach einem der vorherigen Ansprüche 1 bis 5, wobei sich die mobilen Medizinkomponenten (20) in der medizinischen Einrichtung (1) über ein SLAM-Verfahren autonom bewegen.

11. Mobile Medizinkomponente (20) aus einer Menge an mobilen Medizinkomponenten zur kooperativen Bewegungsüberwachung und/oder Bewegungssteuerung der Menge von mobilen Medizinkomponenten (20) umfassend:
- eine Antriebseinheit (21), die zum Antrieb der mobilen Medizinkomponente (20) ausgebildet ist;
- eine Spannungsversorgung (22), die zur elektrischen Versorgung der einzelnen Komponenten der mobilen Medizinkomponente (20) ausgebildet ist;
- eine Kommunikationsschnittstelle (23), die zum Aufbau einer Kommunikationsverbindung mit weiteren mobilen Medizinkomponenten (20) in einer medizinischen Einrichtung (1) ausgebildet ist;
- eine Sensoreinheit (24) zur Erfassung der Umgebung der mobilen Medizinkomponente (20);
- eine Speichereinheit (25) zur Speicherung des angelernten Bewegungsmodells und/oder von Karten der medizinischen Einrichtung (1); und
- eine Prozessoreinheit (26) zum kooperativen Überwachen und/oder Steuern der Bewegung jeder mobilen Medizinkomponente (20) in der medizinischen Einrichtung (1) und/oder in den Einrichtungseinheiten (11) durch Bereitstellen von Steuersignalen zum Ansteuern der Antriebseinheit (21).

12. Verfahren zum Betreiben eines Systems nach einem der Ansprüche 1 bis 10 durch Bereitstellen von lokal und autonom auf der medizinischen Komponente erzeugten Steuersignalen zur kooperativen Bewegungsüberwachung und/oder Bewegungssteuerung der Menge an mobilen Medizinkomponenten in einer Einrichtung (1) und/oder in einer Vielzahl an Einrichtungseinheiten (11) der Einrichtung (1).

13. Verfahren zum Betreiben einer mobilen Medizinkomponente (20) einer Menge an mobilen Medizinkomponenten (20) in einer medizinischen Einrichtung (1) umfassend eine Antriebseinheit (21), eine Spannungsversorgung (22), eine Kommunikationsschnittstelle (23), eine Sensoreinheit (24), eine Speichereinheit (25) und eine Prozessoreinheit (26), mit den Schritten:
- Aktivieren (S1) der Kommunikationsschnittstelle (23) zum kontinuierlichen Empfangen von Navigationssignalen von jeder der mobilen Medizinkomponenten (20) der Menge an mobilen Medizinkomponenten (20) in der medizinischen Einrichtung (1);
- Empfangen (S2) von Navigationssignalen von jeder der mobilen Medizinkomponenten (20);
- Erzeugen (S3) von Steuersignalen für die Antriebseinheit zur Bewegung der mobilen Medizinkomponente (20) auf Basis der empfangenen Navigationssignale und/oder eines Bewegungsmodells und/oder eines Anforderungssignals;
- Erzeugen (S4) von Navigationssignalen über ein Kommunikationsprotokoll auf Basis der erzeugten Steuersignale; und
- Senden (S5) der erzeugte Navigationssignale über die Kommunikationsschnittstelle (23) zur kooperativen Überwachung und/oder Steuerung jeder mobilen Medizinkomponente (20) der Vielzahl an mobilen Medizinkomponenten (20) in der medizinischen Einrichtung (1) und/oder in den Einrichtungseinheiten (11) .

14. Computerprogramm mit Programmcode für das Ausführen eines Verfahrens nach Verfahrensanspruch 13, wenn das Computerprogramm auf einem elektronischen Gerät ausgeführt wird.
